# EUROPEAN PATENT APPLICATION

(11) **EP 1 442 721 A1**
(43) Date of publication of application: **04.08.2004**
(21) Application number: 03029350.0
(22) Date of filing: 19.12.2003
(51) Int. Cl.: A61B 19/02

(54) **Surgical tray with tubing management feature**

(30) Priority: 27.01.2003 US 442927 P; 03.04.2003 US 406404
(71) Applicant: Alcon Inc., 6331 Hunenberg (CH)
(72) Inventor: Buczek, Mark J., Oceanside CA 92054 (US)
(74) Representative: Curley, Donnacha John

(57) **Abstract**

A movable surgical tray (10) is provided, that is connected to a surgical console (100) in use, and contains a troughs or troughs (14) into which various surgical handpiece fluid tubings may be placed. The troughs (4) allow the fluid tubings to move horizontally outwardly from the tray (10), but help to prevent the tubings from sagging or drooping and thereby contacting non-sterile surfaces, when the tray (10) is attached in use to a surgical console (100).

## Description

This application claims the benefit of U.S Provisional Patent Application No. 60/442,927, filed January 27, 2003.

### Background of the Invention

This invention relates generally to the field of ophthalmic surgery and more particularly to surgical trays used with surgical consoles.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the comea, vitreous and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

Alternatively, disease or trauma may affect the retina or vitreous, in many cases requiring that the vitreous be removed.

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is usually replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly.

With respect to vitreous and/or retinal surgery, a variety of cutting devices, scissors, extrusion needles (cannulas), fragmenters or tissue manipulators may be used. Some of these devices, such as vitreous cutters, use a guillotine (axial) or reciprocating hollow cutting tube. Suction is applied to the interior of the cutting tube so that the tissue is aspirated away as it is cut.

The various tubings connecting the various handpieces to the surgical console all must enter the sterile field, and so the tubings themselves must be kept sterile. Prior to the present invention, the tubings were connected to the surgical console and allowed to droop loosely between the console and handpiece, where the tubings could contact non-sterile surfaces and become contaminated. For example, see FIG. 1 in U.S. Patent No. 4,813,927, the entire contents of which being incorporated herein by reference. One prior art method of addressing this concern was to use a sterilized clip to clamp the tubings to the surgical tray. Such clamping of the tubings, however, potentially compromises the fluidic performance of the tubings.

Therefore, a need continues to exist for a device that helps assist at keeping surgical tubings within the sterile field.

### Brief Summary of the Invention

The present invention improves upon the prior art by providing a movable surgical tray that is connected to the surgical console and contains a trough or troughs into which the various handpiece fluid tubing may be placed. The troughs allow the fluid tubings to move horizontally outwardly from the tray, but help prevent the tubings from sagging or drooping and contacting non-sterile surfaces.

Accordingly, one objective of the present invention is to provide a surgical tray having a trough.

Another objective of the present invention is to provide a surgical tray that helps prevent handpiece tubings from sagging or drooping and containing non-sterile surfaces.

These and other advantages and objectives of the present invention will become apparent from the detailed description and claims that follow.

### Brief Description of the Drawings

FIG. 1 is a perspective view of the surgical tray of the present invention being used with a surgical console.
FIG. 2 is a top plan view of the surgical tray of the present invention.
FIG. 3 is a perspective view of the surgical tray of the present invention.

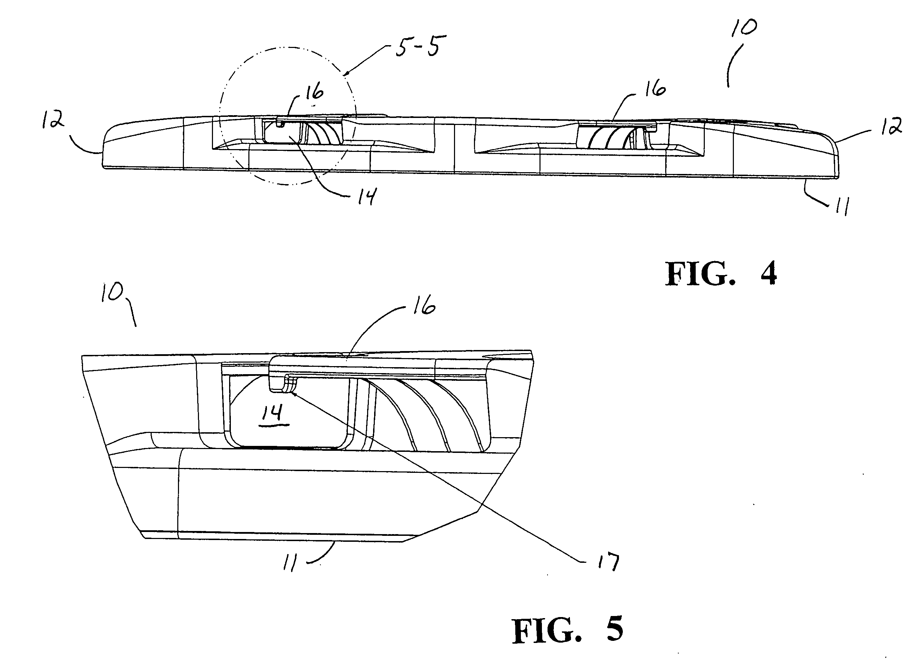

FIG. 4 is an enlarged front elevational view of the surgical tray of the present invention.
FIG. 5 is an enlarged partial elevational view of the surgical tray of the present invention taken at circle 5-5 in FIG. 4.

### Detailed Description of the Invention

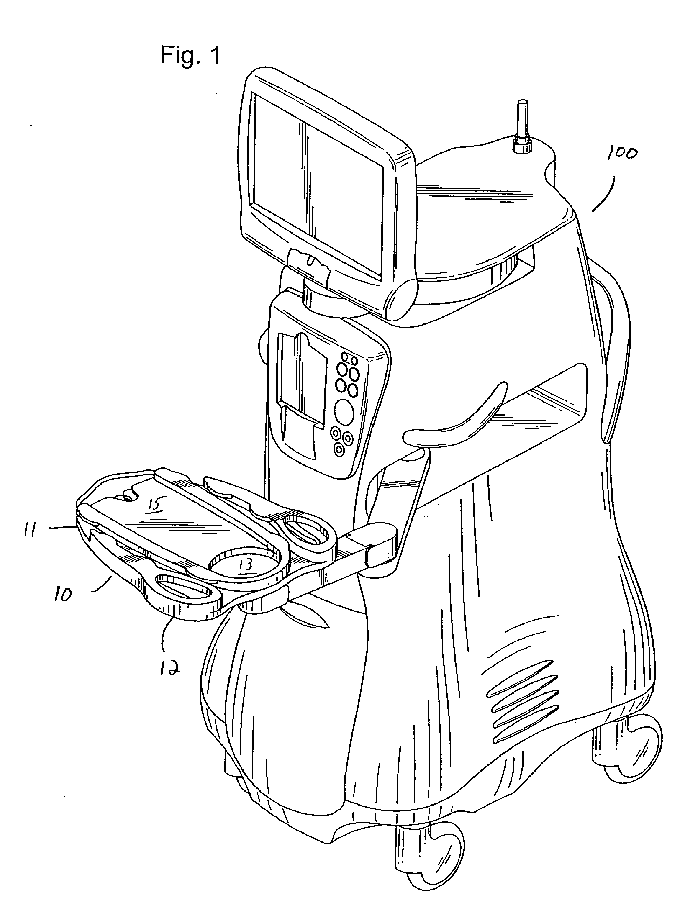

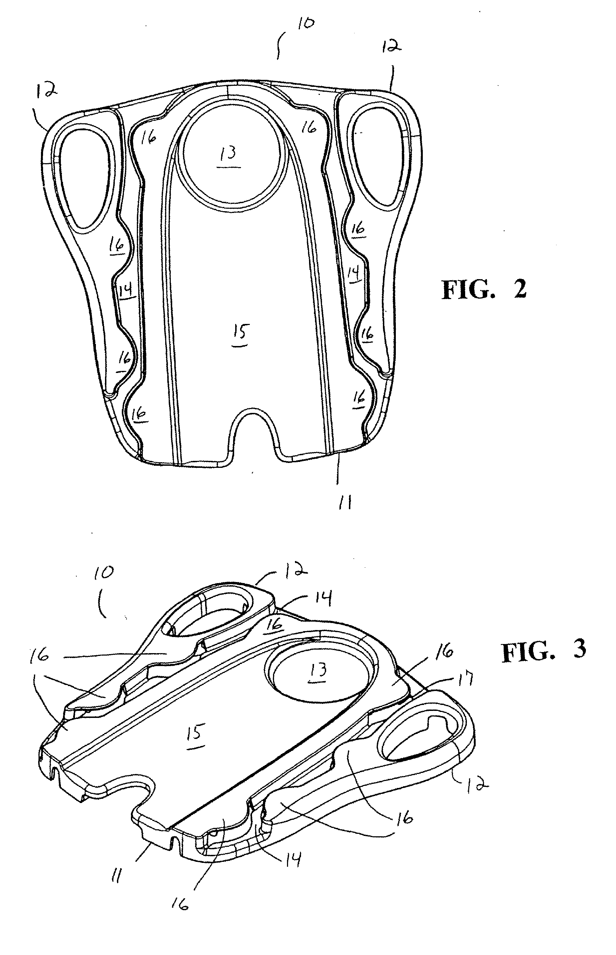

As best seen in FIG. 1, surgical tray 10 of the present invention generally is connected to or forms a part of surgical console 100, such consoles being well-known in the art. For example, U.S. Patent No. Des. 467,001, the entire contents of which being incorporated herein by reference, discloses a surgical console suitable for use with tray 10 of the present invention. As best seen in FIGS. 2 and 3, tray 10 generally contains body 11 that is rectangular in shape with one or more grab handles 12 and a pair of troughs 14. Body 11 may also have one or more recesses 15 for retaining various handpieces and tools used during a surgical procedure and may have recess 13 for retaining a remote control device. Troughs 14 have a plurality of overhanging portions or tabs 16 that partially enclose troughs 14 and are spaced so as to form a tunnel-like passage that helps prevent the tubings (not shown) from being pulled from troughs 14 inadvertently in a vertical direction but still allow for horizontal movement of the tubings. In addition, the underside of tabs 16 may contains retention protrusions 17 which also help hold the tubings within troughs 14. The rounded geometry of tabs 16, however, permit the tubings to be installed and removed from troughs 14 relatively easily when required. Tray 10 is preferably molded in one piece from a suitable thermoplastic.

In use, a sterile surgical drape (not shown) is placed over tray 10. The tubings are then inserted into troughs 14 and maintained within troughs 14 in a sterile condition until the surgical procedure is completed.

This description is given for purposes of illustration and explanation. It will be apparent to those skilled in the relevant art that changes and modifications may be made to the invention described above without departing from its scope.

## Claims

1. A surgical tray (10), comprising:
(a) a body (11);
(b) a plurality of troughs (14) formed in the body;
(c) a plurality of tabs (16) formed in the body, the tabs overhanging the troughs so as to enclose partially the troughs.

2. The surgical tray of claim 1, further comprising a plurality of grab handles (12) formed in the body.

3. The surgical tray of claim 1 or claim 2, wherein the tabs (16) have a rounded geometry in plan view.

4. The surgical tray of any one of claims 1 to 3, wherein the tabs (16) have a retention protrusion (17).

5. The surgical tray of any one of claims 1 to 4, further comprising a recess (13) in the body.

6. The surgical tray of any one of claims 1 to 5, in which the troughs (14) are adapted for placement of surgical handpiece fluid tubings, and adapted for allowing the fluid tubings to move horizontally outwardly from the tray, but help to prevent the tubings from sagging or drooping and thereby contacting non-sterile surfaces, when the tray is attached in use to a surgical console (100).
